# EUROPEAN PATENT SPECIFICATION

(11) **EP 1 109 517 B1**
(45) Date of publication and mention of the grant of the patent: **12.07.2006**
(21) Application number: 99945555.3
(22) Date of filing: 03.09.1999
(51) Int. Cl.: A61F 2/44

(54) **CYLINDRICAL HEMI-LUNAR PARALLEL ARRAY THREADED DISC PROSTHESIS**
MIT PARALLELEM GEWINDE VERSEHENE ZYLINDRISCHE, HALBKUGELFÖRMIGE SCHEIBENPROTHESE
PROTHESE DISCALE, CYLINDRIQUE, HEMISPHERIQUE, A FILETAGES PARALLELES

(30) Priority: 04.09.1998 US 99279 P
(43) Date of publication of application: 27.06.2001
(73) Proprietor: SDGI Holdings, Inc., Memphis, Tennessee 38132 (US)
(72) Inventor: BRYAN, Vincent, Mercer Island, WA 98040 (US); CARVER, Kip, Mercer Island, WA 98040 (US)
(74) Representative: Samuels, Lucy Alice
(86) International application number: PCT/US1999/020459
(87) International publication number: WO 2000/013620

(56) References cited:
- US-A- 4 863 476
- US-A- 5 370 697
- US-A- 5 534 028
- US-A- 5 593 409
- US-A- 5 645 598
- US-A- 5 888 226
- US-A- 5 928 284
- US-A- 5 976 187

## Description

This invention relates to the design and use of a unique disc prosthesis for the lumbar and thoracic spine. By placing one hemi-lunar prosthetic disc within a cylindrical housing of metal, ceramic, or polymeric material, (the housing being separated into two longitudinally separated sections) a small profile prosthesis can be created which will allow placement of the device through a small surgical opening into an intervertebral space within a human spine for which the closest prior art is document US 4 863 476 A. By placing two such cylindrical threaded devices in parallel into the intervertebral space, each housing containing one hemi-lunar resilient disc, positioned so that the convex portions of the two separately housed discs approximate an interrupted circle or toroid, a full range of motion of the functional spinal unit (FSU) can be achieved.

If the hemi-lunar material possesses resilient, viscoelastic properties, and if the housing is split so that the internally placed hemi-lunar disc maintains separation of the upper and lower housing members, a cushioning effect can be obtained.

The hemi-lunar disc material is substantially surrounded by concave surfaces formed within the housing, which are contoured to articulate with the upper and lower surface shapes of the hemi-lunar disc, so that the housings containing the discs may slide and /or rotate over the surface of the hemi-lunar discs to provide for joint space motion.

The exterior surface of the split housing is threaded so that it can be screwed into a pre-tapped hole of appropriate size formed in confronting surfaces of adjacent intervertebral bones or bodies. The upper sectional of the threaded housing engages the cephalad vertebral body's inferior endplate and cancellous bone, and the other section of the threaded housing engages the opposing superior endplate and cancellous bone of the caudal vertebral body when the implant is fully inserted.

Threaded cylindrical housings of differing sizes can be installed in a parallel array so as to allow for appropriate spinal column curvature when inserted into the spine and alined from a lateral direction.

U.S. Patent 5,674,296 shows a spine disc prothesis comprising a relatively supple nuclear central portion.

### BACKGROUND OF THE INVENTION

Degenerative disc disease, including disc herniation, may produce disabling symptoms of local pain, radiculopathy or myelopathy in an otherwise clinically stable spine, which may be unresponsive to non-surgical treatment. Several surgical treatments are available to address the symptoms of degenerative disc disease when non- invasive therapies are not effective. These surgical treatments include decompression, discectomy and fusion. These treatments, and in particular the discectomy and fusion procedures, provide relief of clinical symptoms but they do not restore normal or near normal range of motion or cushioning to the affected functional spinal unit (FSU). This can result in acceleration of the degenerative process in spinal discs adjacent to the original surgical operation site. This degenerative process can, in turn, require additional surgical intervention.

Open surgery and endoscopic techniques are often used to provide access to the intervertebral disc space. Posterior, postero-lateral, and anterior approaches allow placement of instrumentation to facilitate exposure of the degenerated disc and the insertion of bone grafts or fusion cages to accomplish bony fusion.

Because of anatomical structure considerations and instrument size restrictions associated with minimally invasive surgical techniques in the spine, the insertion of a functional disc prosthesis equal in size to the natural disc creates risks due to mechanical interferences with critical vascular structures.

It is the general object of this invention to provide a functional disc prosthesis which provides for a full range of motion of the FSU and for cushioning between two adjacent vertebrae while maintaining stability, intervertebral body spacing and lordosis.

More specifically it is an object of the invention to provide a disc prosthesis having a small or narrow profile. The novel exemplary prosthesis is cylindrical in exterior shape, and is comprised of two longitudinally split halves. Each housing half is separated from the other at all times by a hemi-lunar resilient body contained therein, and is strong enough to support the loads to which it shall be subjected during the activities of daily living. The housing contains a concave articulation recess capable of mating with the hemi-lunar resilient body placed between the two disc halves, and provide geometry which allows sliding and/or rotational motion.

The cylindrical housing is threaded on its exterior for ease of introduction into, and mechanical stability in, a prepared space in the opposing vertebrae of the FSU. The housing is configured to fit the restrictions imposed by the limited anatomical space available for the surgical placement of the implant, and is small so as to allow implantation procedures and instrumentation such as those used in an endoscopic procedure.

It is a further object of the invention to provide cylindrical housings of differing size, and resilient bodies contained within, so that when the cylindrically shaped prostheses units are used in parallel, they facilitate proper positioning of opposing vertebrae.

Another object is to obviate the need for a second surgical site for bone graft harvesting as may be required in spinal fusion procedures.

And it is a further object of the invention to provide a flexible containment barrier or sheath to completely surround and enclose the space occupied by the resilient body between the two cylindrical housing halves, so as to contain lubricant and debris.

Still another object of the invention is to provide a disc prosthesis which will permit motion between the housing halves.

A further object of the intention is to provide a disc prosthesis which will provide for cushioning between the housing halves.

It is a still further object of the invention to provide a disc prosthesis which may be used alone or in parallel array with similar prostheses.

Another object of the invention is to have ports in the housing for the introduction of a lubricant for the nucleus and housing interior. The ports can be sealed with a plug, screw or other device.

Other objects and advantages of the invention will become apparent to those skilled in the art upon reading the following detailed description and upon reference to the drawings. Throughout the drawings, like reference numerals refer to like parts.

### DESCRIPTION OF THE DRAWINGS

Figure 1 is an exploded view of two of the novel prostheses arranged in parallel array.
Figure 2 is a top plan view of the prostheses shown in figure 1.
Figure 3 is a sectional view taken substantially in the plane of line 3 - 3 in figure 2.
Figure 4 is a sectional view taken substantially the plane of line 4 - 4 in figure 3.
Figure 5 is an isometric view of a housing half.
Figure 6 is a top plan view of the housing half shown in figure 5.
Figure 7 is a side elevation view of the housing half shown in figures 5 and 6.
Figure 8 is a sectional view taken substantially in the plane of line 8 -8 in figure 7.
Figure 9 is a developed end view taken substantially in the plane of line 9 - 9 in figure 7.
Figure 10 is a top plan view of the housing half shown in figures 6 and 7.
Figure 11 is a fragmentary view of a portion of the edge of the housing half shown in figure 8.
Figure 12 is a top plan view of an alternative embodiment of the hemi-lunar disc.
Figure 13 is an end view of the alternate embodiment hemi-lunar disc shown in figure 12.

While the invention will be described in connection with preferred embodiments, it will be understood that it is not intended to limit the invention to these embodiments. On the contrary, it is intended to cover all alternatives, modifications and equivalents as may be included within the spirit and scope of the invention as defined by the appended claims.

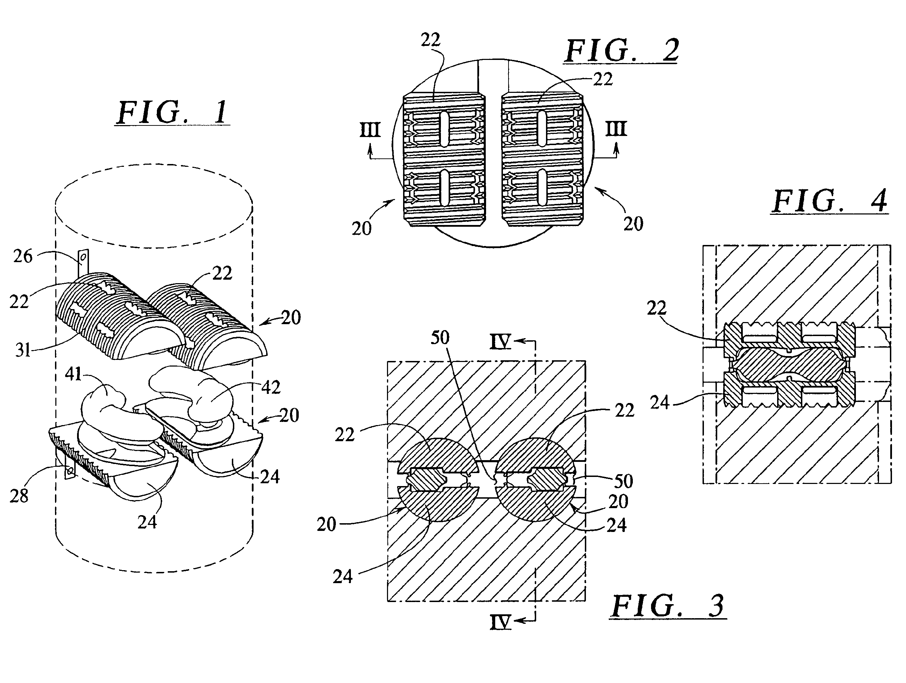

To accomplish the objectives set out above, the novel exemplary disc prosthesis 10 includes, as shown in figures 1 - 4, a cylindrical housing 20. The housing 20 includes an upper half housing 22 and a lower half housing 24. In the embodiment shown in figure 1, an upper fixator wing 26 is welded or otherwise associated with the upper half housing 22, and a lower fixator wing 28 is similarly welded or otherwise associated with the lower half housing 24. These wings 26, 28 can be used to stabilize the prosthesis to bone, as suggested in U.S. Patent 5,674,296. The wings 26, 28 can be omitted, as suggested in figures 2 - 10.

As particularly shown in figures 2 and 4, a hemi-lunar shaped viscoelastic disc 41 is interposed between the upper half housing 22 and the lower half housing 24 to maintain the housing halves separate from one another and to provide for a defined range of motion. The shape of the hemi-lunar disc 41 can be partially bi-convex discoid. The disc provides stability and limitation against excessive motion of the housing halves and prosthesis during motion of the functional spinal unit. The disc can have a relatively soft and resilient interior and a relatively hard end durable exterior. Alternatively, the disc element 41 may be made of a suitable hydrogel.

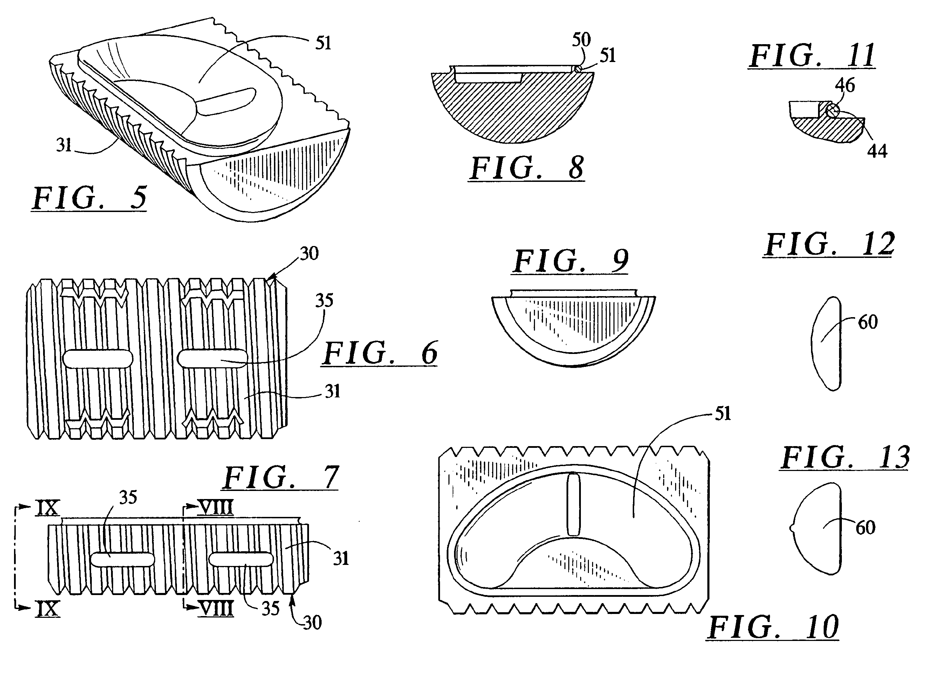

An alternative embodiment of the hemi-lunar disc is shown in figures 12 and 13. To provide additional stability to the implant, this resilient disc 60 takes the general appearance of an elongated hemisphere. Corresponding modifications are made to the interior of the housing halves 22, 24.

When implants 20 are used in parallel array as suggested and figures 1 and 2, the arrangement of these semi-lunar disks 41, 42 resembles an interrupted toroid.

The housing 20 has an exterior surface 30 which bears a screw thread shape 31. This screw thread shape 31 is continuous, and is contiguous from housing half 22 to housing half 24 so that the disc prosthesis can be screwed into a pre-tapped intervertebral space hole formed within between two adjacent vertebrae as, for example, in the human spine. If desired, recesses 35 can be formed to permit bone ingrowth and further stabilization of the device. The housing 20 shape can be that of a right cylinder or it can be conical.

In accordance with one aspect of the invention, this small or thin profile prosthesis 20 can be implanted in the spine through a small surgical opening. One device 20 may be used, or by placing two such devices 20 in parallel as suggested in figures 1 and 2, a full range of motion of the functional spinal unit (FSU) can be achieved. If the discoid material possesses resilient, viscoelastic properties similar to the removed natural disk, with the housing being split or otherwise open on its sides with the internally placed hemi-lunar disc maintaining the separation of the upper and lower housing members 22 and 24, , a cushioning effect may also be realized.

If desired, a flexible sheath or seal 50 can be attached to the housing halves, as by a retaining wire or circlage band 41 tensioned in a groove 46, as suggested in figures 3,8 and 11, and in U.S. Patent 5,674,296.

As suggested in figures 5 and 10, each hemi-lunar disc 41, 42 can be carried in a concave surface 51 formed or contained within the housing, and contoured to accept the upper and lower surface shape of the hemi-lunar disc 41 so that the housings 20 each comprising the two or more halves or paired shells 23, 24 may slide and/or rotate over the surface of the discs 41, 42 to provide for joint space separation and motion.

As noted above, the exterior surface of the split housing 20 has a threaded formation so that it may be screwed into a pre-tapped hole of appropriate size at an intervertebral disc space. When properly screwed into place, the upper half of the threaded housing engages the cephalad vertebral bone inferior endplate and the other half 24 of the threaded housing engages the opposing superior endplate of the caudal vertebral bone when fully inserted.

The device may be inserted via open or minimally invasive techniques including endoscopy, or by a variety of known surgical approaches where adequate anatomical space is available. Though the prosthesis is inserted as a single threaded cylindrical unit, its final position is such that one half of the housing is left exclusively in contact with the endplate and cancellous bone of one vertebral body and the other in the exclusive contact of the opposing vertebral body endplate and cancellous bone. The hemi-lunar resilient bodies between the cylindrical housing halves allow movement by providing for sliding and rotating in multiple directions and cushioning in response to physiological loads placed upon them. When housing cylinders of different size are at used in parallel, intervertebral spacing can be varied to achieve desired vertebrae positional relationships.

By varying the shapes of the housings, and the contained sliding discs, a non-parallel array can be utilized, especially in cases of significant anatomical interferences.

## Claims

1. A disc prosthesis for insertion into an intervertebral space between two vertebral end plates, the disc prosthesis comprising, in combination: a cylindrical housing of a conical housing (20), said housing defining an axis wherein the housing is insertable into the intervertebral space between the end plates so that the axis is generally aligned with at least one of the end plates, the housing including an upper half (22) and a lower half (24) a disc (41, 42, 60) interposed between the upper half housing (22) and the lower half housing (24) to maintain the housing halves separate from one another, **characterised in that** said disc is a resilient, viscoelastic disc.

2. A disc prosthesis according to claim 1 wherein said disc (41, 42, 60) is hemi-lunar in shape.

3. A disc prosthesis according to claim 1 wherein the disc (41, 42, 60) is partly surrounded by a concave surface formed within said housing.

4. A disc prosthesis according to claim 1 wherein said housing (20) has a threaded exterior surface (30) bearing a screw thread shape (31).

5. A disc prosthesis according to claim 4 wherein said screw thread (31) is continuous, and is contiguous from housing half to housing half so that the disc prosthesis can be screwed into a pre-tapped intervertebral space hole.

6. A disc prosthesis according to claim 1 wherein recesses (35) are defined in said housing to permit bone ingrowth.

7. A disc prosthesis according to claim 1 including a wing member (26, 28) attached to each of the upper and the lower half housing members, the wings permitting the housing halves to be affixed to spinal vertebrae.

8. A disc prosthesis according to claim 1 having recesses (35) defined in its exterior surface to permit bone ingrowth.

9. A disc prosthesis according to claim 1 for use in a human spinal implant wherein the viscoelastic disc has convex external surfaces for sliding engagement with concave surfaces formed on the interior of rigid upper and lower half housings (22, 24).

10. The disc of claim 9 wherein said disc has a relatively soft and resilient interior and a relatively hard and durable exterior.

11. A disc prosthesis according to claim 1 wherein the housing halves (22, 24) include an exterior surface defining a general continuous thread formation (31), and the viscoelastic disc (41, 42, 60) is hemi-lunar and is interposed between the housing halves to maintain the housing parts separate from one another but to provide cushioning between the housing halves and to permit limited motion between the housing halves.

12. A disc prosthesis according to claim 11 wherein the housing thread (31) is adapted to engage the bone of adjacent vertebral bodies.

13. A disc prosthesis according to claim 11 including a sheath (50) attached to said housing halves.

14. A disc prosthesis according to claim 11, the disc (41, 42, 60) having a partial bi-convex discoid shape.

15. A disc prosthesis according to claim 1, including a sealable portal for introduction of a lubricant.

16. A disc prosthesis according to claim 1, further including a sheath (50) interconnecting the housing halves.

17. A disc prosthesis according to claim 1, including a second cylindrical or conical housing, the second housing being insertable within an intervertebral disc space adjacent the first cylindrical or conical housing, the second housing including a second upper half and a second lower half and a second resilient, viscoelastic disc interposed between the second upper and lower half housings to maintain the second housing halves separate from one another.

18. A disc prosthesis according to claim 1, wherein the disc is insertable within less than half of the intervertebral space.

19. A system for implantation into a vertebral space comprising:
the disc prosthesis according to claim 18, wherein the disc prosthesis is a first disc prosthesis; and
a second disc prosthesis according to claim 18, wherein the first and second prosthetic discs are insertable into the same vertebral space.

## Patentansprüche

1. Scheibenprothese zur Einfügung in einen intervertebralen Raum zwischen zwei vertebralen Endplatten, wobei die Scheibenprothese in Kombination umfasst: ein zylindrisches Gehäuse oder ein konisches Gehäuse (20), wobei das Gehäuse eine Achse definiert, wobei das Gehäuse in den intervertebralen Raum zwischen den Endplatten einfügbar ist, so dass die Achse im Allgemeinen mit zumindest einer der Endplatten ausgerichtet ist, wobei das Gehäuse eine obere Hälfte (22) und eine untere Hälfte (24) aufweist, wobei eine Scheibe (41, 42, 60) zwischen der oberen Hälfte des Gehäuses (22) und der unteren Hälfte des Gehäuses (24) angeordnet ist, um die Gehäusehälften voneinander getrennt zu halten, **dadurch gekennzeichnet, dass** die Scheibe eine nachgiebige, viskoelastische Scheibe ist.

2. Scheibenprothese gemäß Anspruch 1, bei der die Scheibe (41, 42, 60) halbmondförmig ist.

3. Scheibenprothese gemäß Anspruch 1, bei der die Scheibe (41, 42, 60) teilweise von einer in dem Gehäuse ausgebildeten konkaven Oberfläche umgeben ist.

4. Scheibenprothese gemäß Anspruch 1, bei der das Gehäuse (20) eine äußere Gewindeoberfläche (20) aufweist, die eine Schraubengewindeform (31) trägt.

5. Scheibenprothese gemäß Anspruch 4, bei der das Schraubengewinde (31) kontinuierlich und von Gehäusehälfte zu Gehäusehälfte zusammenhängend ist, so dass die Scheibenprothese in ein intervertebrales Raumloch mit Innengewinde geschraubt werden kann.

6. Scheibenprothese gemäß Anspruch 1, bei der Ausnehmungen (35) in dem Gehäuse definiert sind, um Knocheneinwuchs zu erlauben.

7. Scheibenprothese gemäß Anspruch 1, umfassend ein Flügelelement (26, 28), das an jedem der oberen und unteren Hälften der Gehäuseelemente befestigt ist, wobei die Flügel den Gehäusehälften ermöglichen, an Rückenwirbeln angebracht zu werden.

8. Scheibenprothese gemäß Anspruch 1, die in ihrer äußeren Oberfläche definierte Ausnehmungen (35) aufweist, um Knocheneinwuchs zu erlauben.

9. Scheibenprothese gemäß Anspruch 1, zur Verwendung in einem menschlichen spinalen Implantat, wobei die viskoelastische Scheibe konvexe äußere Oberflächen zur verschiebbaren Ineingriffnahme mit konkaven Oberflächen aufweist, die auf dem Inneren von festen oberen und unteren Gehäusehälften (22, 24) ausgebildet sind.

10. Scheibe gemäß Anspruch 9, bei der die Scheibe ein relativ weiches und nachgiebiges Inneres und ein relativ hartes und haltbares Äußeres aufweist.

11. Scheibenprothese gemäß Anspruch 1, bei der die Gehäusehälften (22, 24) eine äußere Oberfläche aufweisen, die eine allgemeine kontinuierliche Gewindeformation (31) definiert, und die viskoelastische Scheibe (41, 42, 60) halbmondförmig ist und zwischen den Gehäusehälften angeordnet ist, um die Gehäuseteile getrennt voneinander zu halten, jedoch eine Abfederung zwischen den Gehäusehälften bereitzustellen, und um eine begrenzte Bewegung zwischen den Gehäusehälften zu erlauben.

12. Scheibenprothese gemäß Anspruch 11, bei der das Gehäusegewinde (31) angepasst ist, um den Knochen von angrenzenden vertebralen Körpern in Eingriff zu nehmen.

13. Scheibenprothese gemäß Anspruch 11, umfassend eine Hülle (50), die an den Gehäusehälften befestigt ist.

14. Scheibenprothese gemäß Anspruch 11, wobei die Scheibe (41, 42, 60) eine teilweise bikonvexe Scheibenform aufweist.

15. Scheibenprothese gemäß Anspruch 1, umfassend einen abdichtbaren Eingang zur Einführung eines Schmiermittels.

16. Scheibenprothese gemäß Anspruch 1, ferner umfassend eine Hülle (50), die die Gehäusehälften untereinander verbindet.

17. Scheibenprothese gemäß Anspruch 1, umfassend ein zweites zylindrisches oder konisches Gehäuse, wobei das zweite Gehäuse in einen intervertebralen Scheibenraum angrenzend dem ersten zylindrischen oder konischen Gehäuse einfügbar ist, wobei das zweite Gehäuse eine zweite obere Hälfte und eine zweite untere Hälfte und eine zweite nachgiebige viskoelastische Scheibe aufweist, die zwischen den zweiten oberen und unteren Gehäusehälften angebracht ist, um die zweiten Gehäusehälften getrennt voneinander zu halten.

18. Scheibenprothese gemäß Anspruch 1, bei der die Scheibe in weniger als die Hälfte des intervertebralen Raums einfügbar ist.

19. System zur Implantation in einen vertebralen Raum, mit:
der Scheibenprothese gemäß Anspruch 18, bei der die Scheibenprothese eine erste Scheibenprothese ist; und
einer zweiten Scheibenprothese gemäß Anspruch 18, bei der die ersten und zweiten Prothesescheiben in den gleichen vertebralen Raum einfügbar sind.

## Revendications

1. Prothèse discoïde pour l'insertion dans un espace intervertébral entre deux plaques terminales de vertèbre, la prothèse discoïde comprenant, en combinaison : un boîtier cylindrique ou un boîtier conique (20), ledit boîtier définissant un axe où le boîtier peut être inséré dans l'espace intervertébral entre les plaques terminales de sorte que l'axe est généralement aligné avec au moins une des plaques terminales, le boîtier comprenant une moitié supérieure (22) et une moitié inférieure (24), un disque (41, 42, 60) interposé entre le boîtier de moitié supérieure (22) et le boîtier de moitié inférieure (24) pour maintenir les demi-boîtiers séparés l'un de l'autre, **caractérisé en ce que** ledit disque est un disque résilient, viscoélastique.

2. Prothèse discoïde selon la revendication 1, dans laquelle ledit disque (41, 42, 60) est en forme de demi-lune.

3. Prothèse discoïde selon la revendication 1, dans laquelle le disque (41, 42, 60) est partiellement entouré d'une surface concave formée par ledit boîtier.

4. Prothèse discoïde selon la revendication 1, dans laquelle ledit boîtier (20) a une surface extérieure filetée (30), portant une forme de filet de vis (31).

5. Prothèse discoïde selon la revendication 4, dans laquelle ledit filet de vis (31) est continu, et contigu d'un demi-boîtier à un demi-boîtier de sorte que la prothèse discoïde peut être vissée dans un trou d'espace intervertébral pré-taraudé.

6. Prothèse discoïde selon la revendication 1, dans laquelle des évidements (35) sont définis dans ledit boîtier pour permettre la croissance osseuse.

7. Prothèse discoïde selon la revendication 1, comprenant un élément de type aile (26, 28), attaché à chacun des éléments de boîtier supérieur et de boîtier inférieur, les ailes permettant aux demi-boîtiers d'être fixés aux vertèbres spinales.

8. Prothèse discoïde selon la revendication 1, ayant des évidements (35), définis dans sa surface extérieure pour permettre la croissance osseuse.

9. Prothèse discoïde selon la revendication 1, à utiliser dans un implant vertébral humain, où le disque viscoélastique a des surfaces externes convexes pour un engagement par glissement avec les surfaces concaves formées sur l'intérieur des demi-boîtiers supérieur et inférieur rigides (22, 24).

10. Disque selon la revendication 9, où le disque a un intérieur relativement souple et résilient et un extérieur relativement dur et durable.

11. Prothèse discoïde selon la revendication 1, dans laquelle les demi-boîtiers (22, 24) comprennent une surface extérieure définissant une formation générale de filet continu (31) et le disque viscoélastique (41, 42, 60) est en forme de demi-lune et est interposé entre les demi-boîtiers pour maintenir les parties de boîtier séparées l'une de l'autre, mais pour procurer un amortissement entre les demi-boîtiers et pour permettre un déplacement limité entre les demi-boîtiers.

12. Prothèse discoïde selon la revendication 11, dans laquelle le filet de boîtier (31) est adapté pour engager l'os des corps vertébraux adjacents.

13. Prothèse discoïde selon la revendication 11, comprenant une gaine (50) attachée aux dits demi-boîtiers.

14. Prothèse discoïde selon la revendication 11, le disque (41, 42, 60) ayant une forme discoïde biconvexe partielle.

15. Prothèse discoïde selon la revendication 1, comprenant un portail scellable pour l'introduction d'un lubrifiant.

16. Prothèse discoïde selon la revendication 1, comprenant de plus, une gaine (50), interconnectant les demi-boîtiers.

17. Prothèse discoïde selon la revendication 1, comprenant un deuxième boîtier cylindrique ou conique, le deuxième boîtier étant insérable dans un espace de disque intervertébral adjacent au premier boîtier cylindrique ou conique, le deuxième boîtier comprenant un deuxième demi-boîtier supérieur et un deuxième demi-boîtier inférieur et un deuxième disque viscoélastique, résilient interposé entre les deuxièmes demi-boîtiers inférieur et supérieur pour maintenir les deuxièmes demi-boîtiers séparés l'un de l'autre.

18. Prothèse discoïde selon la revendication 1, dans laquelle le disque peut être inséré dans moins de la moitié de l'espace intervertébral.

19. Système d'implantation dans l'espace intervertébral, comprenant : la prothèse discoïde selon la revendication 18, où la prothèse discoïde est une première prothèse discoïde, et
une deuxième prothèse discoïde selon la revendication 18, où les premiers et deuxièmes disques prothétiques sont insérables dans le même espace intervertébral.
